# EUROPEAN PATENT APPLICATION

(11) **EP 2 394 585 A1**
(43) Date of publication of application: **14.12.2011**
(21) Application number: 11169124.2
(22) Date of filing: 08.06.2011
(51) Int. Cl.: A61B 17/04, A61B 17/00

(54) **Systems for suture passage**

(30) Priority: 02.07.2010 FR 1055393; 10.06.2010 US 353650 P
(71) Applicant: Tornier, Inc., Edina, MN 55435 (US)
(72) Inventor: Snell, Douglas, Amesbury, Massachusetts 01913 (US); Morgan, Daniel E., Salem, Massachusetts 01970 (US)
(74) Representative: Grand, Guillaume

(57) **Abstract**

The invention relates to a system for suture passage through a tissue comprising a suture snare (150), comprising a suture capture element (154); and a piercing tip (108; 192) configured to form through the tissue a hole connecting a first side and a second side of the tissue. According to the invention, the suture snare further comprises a resilient anchor element (152), which has, in an undeformed state of the resilient anchor element, at least one dimension larger than a largest dimension of the hole, and which is configured to deform during insertion of the resilient anchor element through the hole from the first side to the second side of the tissue, then to substantially resume, after passing through the hole, the undeformed state that holds the resilient anchor element (152) on the second side of the tissue.

## Description

The present invention relates to a system for passing a suture. The invention relates generally to suture passing, and more specifically to systems and methods for releasably grasping and passing through tissue a suture snare.

Suture passing in the gleno-humeral space during instability repair is often difficult due to the limited accessibility of the space, the location of implants and sutures around the glenoid rim, and the common requirement of both re-attaching the labrum and plication of the tendon capture. Current techniques use two instruments to pass and retrieve sutures, which requires a second portal to the gleno-humeral space. An example is provided by WO-A-2007/073931.

Similar challenges are experienced in other types of surgeries, particularly arthroscopic surgeries. Thus, WO-A-2007/019374, on which is based the preamble of claim 1, discloses an implant passer comprising a piercing tip from which a snare intended to capture an implant is movable. However, this device is specifically designed to capture an implant after having pierced a tissue by the tip, then to drive this implant through the tissue, in an opposite direction of the prior piercing. The device cannot be used to efficiently put in place an implant on the exit side of a tissue which is pierced by the tip, because the implant tends to be driven with the tip when the tip and the snare, which is integrated into the tip, are withdrawn in an opposite direction of the insertion thereof.

It is an object of the present invention to propose a system for suture passage, that is more efficient and easier to use.

To this end, the subject of the invention is a system according to claim 1. Additional advantageous features of this system are recited in dependent claims 2 to 15.

Embodiments of the present invention permit an ability to continually sew or stitch bites of tissue together by passing a suture snare through the tissue, then picking it up on the other side of the tissue with the same device. This allows for single portal, single handed suture passage in a repeatable pattern, completing each suture path in one evolution, according to embodiments of the present invention. Embodiments of the present invention permit reattachment of a suture snare *in vivo* after deploying the suture snare through tissue and optionally making multiple suture passes before (or after) attaching the suture to the snare, and pulling both snare and suture through tissue.

Embodiments of the present invention permit snare attachment to a suture passer which can be deployed and removably attached to the tissue, then reattached *in vivo,* permit an ability to make several tissue passes in the same evolution, and/or permit single-portal suture passing and retrieval, with a one-handed device.

A system for suture passage through a tissue according to one embodiment of the present invention includes a suture snare, the suture snare including a resilient anchor element and a suture capture element, a passer tube, wherein the passer tube is substantially rigid, an actuator rod, a snare enclosure formed by the passer tube and the actuator rod, wherein the actuator rod is movable to move the snare enclosure between a closed configuration in which the snare enclosure completely captures a portion of the suture snare and an open configuration in which the snare enclosure does not completely capture the portion of the suture snare and permits release of the suture snare from the snare enclosure, and a piercing tip configured to form a hole through the tissue, wherein at least one dimension of the resilient anchor element in an undeformed state of the resilient anchor element is larger than a largest dimension of the hole, and wherein the resilient anchor element is configured to deform during insertion of the resilient anchor element through the hole by the snare enclosure and to substantially resume the undeformed state after passing through the hole.

A method for suture passage through a tissue, which can be implanted by an embodiment of the present invention, includes capturing at least a portion of a suture snare with a snare enclosure, the snare enclosure formed by a passer tube and an actuator rod, wherein the actuator rod is movable to move the snare enclosure between a closed configuration in which the snare enclosure completely captures the portion of the suture snare and an open configuration in which the snare enclosure does not completely capture the portion of the suture snare and permits release of the suture snare from the snare enclosure, piercing the tissue from a first tissue side to a second tissue side to form a hole, wherein the suture snare comprises a resilient anchor element and a suture capture element, and wherein at least one dimension of the resilient anchor element in an undeformed state of the resilient anchor element is larger than a largest dimension of the hole, passing the resilient anchor element through the hole from the first tissue side to the second tissue side by passing the snare enclosure through the hole and by deforming the resilient anchor element, releasing the portion of the suture snare from the snare enclosure by moving the actuator rod to place the snare enclosure into the open configuration, moving the actuator rod to place the snare enclosure into the closed configuration, and passing the snare enclosure back through the hole from the second tissue side to the first tissue side as the undeformed state of the resilient anchor element holds the resilient anchor element on the second tissue side.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.
FIG. 1 illustrates a perspective view of a suture passer device, according to embodiments of the present invention.
FIG. 2 illustrates an exploded perspective view of the suture passer device of FIG. 1, according to embodiments of the present invention.
FIG. 3 illustrates a partial longitudinal cross section view of the suture passer device handle of FIGS. 1 and 2, according to embodiments of the present invention.
FIG. 4 illustrates a perspective view of a piercing tip, according to embodiments of the present invention.
FIG. 5 illustrates a front view of the piercing tip of FIG. 4, according to embodiments of the present invention.
FIG. 6 illustrates a side view of the piercing tip of FIGS. 4 and 5, according to embodiments of the present invention.
FIG. 7 illustrates a top view of the piercing tip of FIGS. 4-6, according to embodiments of the present invention.
FIG. 8 illustrates a bottom view of the piercing tip of FIGS. 4-7, according to embodiments of the present invention.
FIG. 9 illustrates a cross sectional view of the piercing tip of FIGS. 4-8, taken along line C-C of FIG. 6, according to embodiments of the present invention.
FIG. 10 illustrates a suture snare, according to embodiments of the present invention.
FIG. 11 illustrates a resilient anchor element of a suture snare captured by a snare enclosure formed by a piercing tip and a passer tube, with the snare enclosure in a closed configuration, according to embodiments of the present invention.
FIG. 12 illustrates a resilient anchor element released from the snare enclosure with the snare enclosure in an open configuration, according to embodiments of the present invention.
FIG. 13 illustrates a snare enclosure retracted back into the closed position after release of the resilient anchor element, according to embodiments of the present invention.
FIG. 14 illustrates a resilient anchor element of a suture snare being passed through labrum tissue by a suture passer with the resilient anchor element captured by a snare enclosure of the suture passer, according to embodiments of the present invention.
FIG. 15 illustrates a suture passer with the snare enclosure in an open configuration for hooking or capturing the resilient anchor element of the suture snare, according to embodiments of the present invention.
FIG. 16 illustrates an optional passage of the suture passer through capsule tissue and subsequent capture of the resilient anchor element by the snare enclosure of the suture passer, according to embodiments of the present invention.
FIG. 17 illustrates the suture snare being pulled through the labrum and the capsule, pulling the suture along therewith, according to embodiments of the present invention.
FIG. 18 illustrates an alternative suture passer and snare enclosure configuration, according to embodiments of the present invention.
FIG. 19 illustrates a piercing tip and a passer tube forming a snare enclosure in a closed configuration, according to embodiments of the present invention.
FIG. 20 illustrates the piercing tip and the passer tube of FIG. 19 forming a snare enclosure in an open configuration, according to embodiments of the present invention.
FIG. 21 illustrates a piercing tip and passer tube forming an alternative snare enclosure in a closed configuration, according to embodiments of the present invention.
FIG. 22 illustrates the piercing tip and passer tube of FIG. 21 forming the alternative snare enclosure in an open configuration, according to embodiments of the present invention.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

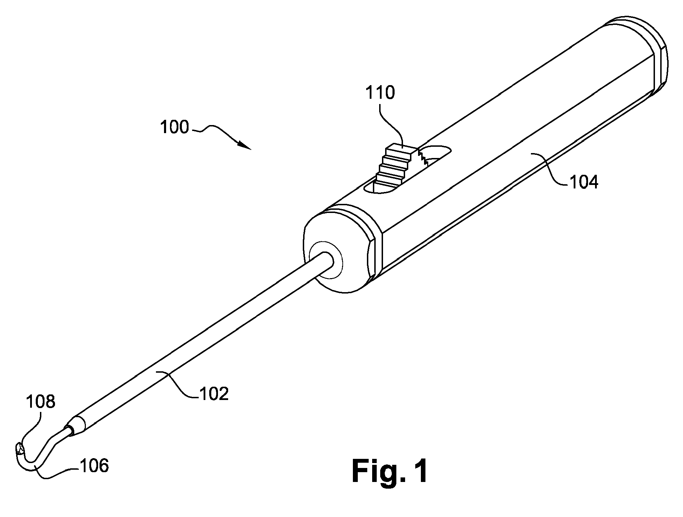

FIG. 1 illustrates a perspective view of a suture passer device 100, according to embodiments of the present invention. Suture passer 100 includes a passer tube 102 and a handle portion 104, according to embodiments of the present invention. The handle portion 104 includes an actuator button 110, and the passer tube 102 includes a curved portion 106. Suture passer 100 also includes a piercing tip 108, according to embodiments of the present invention.

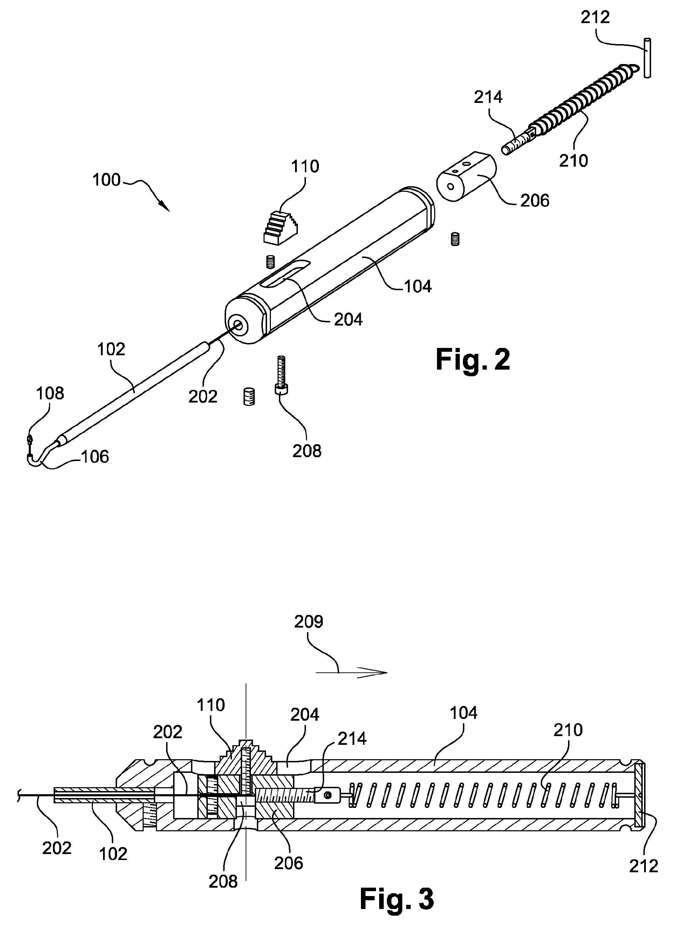

FIG. 2 illustrates an exploded perspective view of the suture passer device 100 of FIG. 1, according to embodiments of the present invention. FIG. 3 illustrates a partial longitudinal cross section view of the suture passer device handle 104 of FIGS. 1 and 2, according to embodiments of the present invention. Button 110, which may also be referred to as a switch, is coupled to slider 206 by screw 208, according to embodiments of the present invention. As used herein, the term "coupled" is used in its broadest sense to refer to elements which are connected, attached, and/or engaged, either directly or integrally or indirectly via other elements, and either permanently, temporarily, or removably.

The slider 206 is coupled to an actuator rod 202 which extends within and slides with respect to the passer tube 102, according to embodiments of the present invention. The actuator rod 202 may be formed of, for example, 0,365 mm (0.025 inch) diameter wire-rod. The handle 104 may further include a biasing element, such as, for example, a tension spring 210. Tension spring 210 may be coupled with a slider attachment mechanism 214 on one end and to an end stop 212 on the other end, according to embodiments of the present invention. Spring 210 thus biases the slider 206 in a direction indicated by arrow 209, according to embodiments of the present invention. According to embodiments of the present invention, the forward and backward motion of slider 206 is limited by the front and back extents of the slot 204. Although FIGS. 2 and 3 depict a particular handle and/or actuator rod 202 actuation mechanism, one of ordinary skill in the art, based on the disclosure provided herein, will recognize the numerous other handle configurations and actuator rod 202 actuation mechanisms that may be used to move the actuator rod 202 with respect to the passer tube 102.

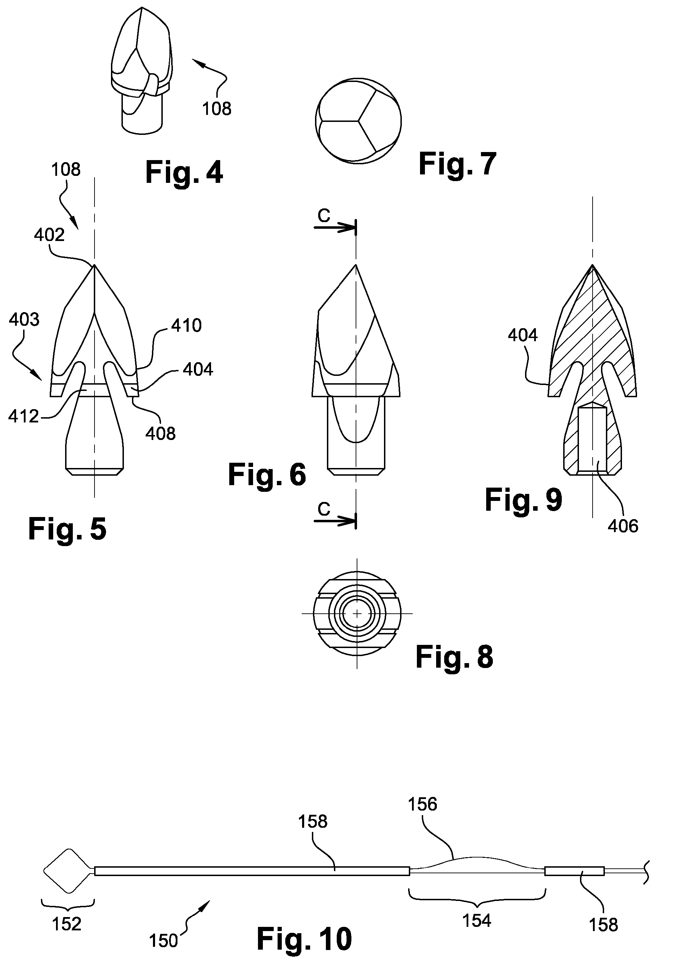

FIGS. 4 through 9 illustrate a piercing tip 108 according to embodiments of the present invention. Piercing tip 108 includes a tip 402 which may be very sharp to easily penetrate tissue and/or form a hole through tissue, according to embodiments of the present invention. Piercing tip 108 may also include one or more hook elements 403 formed by downwardly-hanging flanges 404. The piercing tip 108 may also include a bore 406 formed at its lower end; the bore 406 may be configured for coupling with the distal end of the actuator rod 202, according to embodiments of the present invention. The actuator rod 202 may be rigidly and/or permanently coupled to the piercing tip 108, such that moving the actuator rod 202 imparts a corresponding movement to the piercing tip 108, according to embodiments of the present invention.

FIG. 10 illustrates a suture snare 150, according to embodiments of the present invention. Suture snare includes a resilient anchor element 152 and a suture capture element 154, according to embodiments of the present invention. The resilient anchor element 152 and/or the suture capture element 154 may be formed from one or more wires 156, for example one or more 0,254 mm (0.010 inch) diameter nitinol shape set wires, according to embodiments of the present invention. For example, the suture snare 150 may be comprised of a shape memory wire folded once, which is formed at one end as a resilient anchor element 152, and which includes one or more sheath or coating elements 158 between the resilient anchor element 152 and the suture capture element 154 and/or between the suture capture element 154 and the opposite end of the suture snare 150, according to embodiments of the present invention.

The resilient anchor element 152 may include shape memory or shape set properties, such that it can be deformed (for example bent, twisted, compressed) while returning to its undeformed state after deformation, according to embodiments of the present invention. The resilient anchor element 152 may be configured to have a square, rectangle, or diamond shape, for example the shape illustrated in FIG. 10, in its undeformed, or normal, state. The resilient anchor element 152 may include one or more angular bends (as opposed to curves or continuous curvature), for example, one, two, three, four, or more angular bends, to further emphasize the anchor element's 152 return to its original shape after deformation, and to provide enhanced anchoring capabilities, according to embodiments of the present invention. The resilient anchor element 152 may be configured to have other shapes; for example, the resilient anchor element 152 may be round, and/or may be formed in the shape of a circle or oval or semi-circle, according to embodiments of the present invention.

According to embodiments of the present invention, the resilient anchor element 152 is substantially two-dimensional, in other words, is substantially coplanar. For example, the square-shape of the resilient anchor element 152 shown in FIG. 10 may extend in substantially the same plane. According to other embodiments of the present invention, the resilient anchor element 152 comprises a three-dimensional shape. For example, the square-shaped resilient anchor element 152 shown in FIG. 10 may include a bend or wave or J-shape or other three-dimensional feature apparent from a different view. The resilient anchor element 152 may take numerous other three-dimensional shapes, such as, for example, a box, a rectangular frame, a sphere, a ball, a double helix, a pyramid, and/or a horseshoe. One of ordinary skill in the art, based on the present disclosure, will recognize numerous other three-dimensional shapes that may be imparted to resilient anchor element 152.

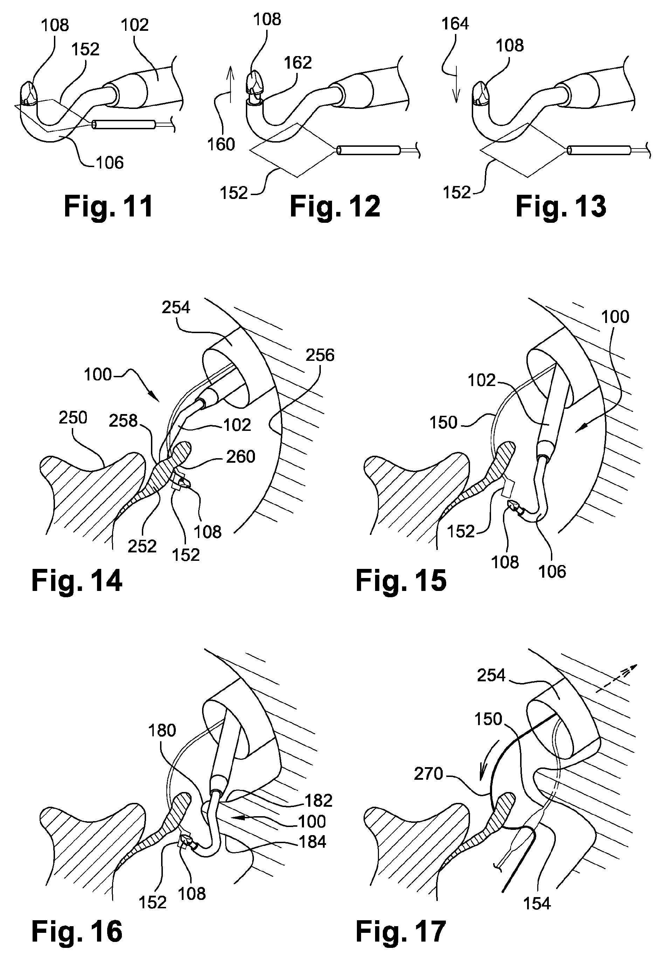

FIGS. 11 through 13 illustrate a suture snare 150 attachment and release process, according to embodiments of the present invention. FIG. 11 illustrates a resilient anchor element 152 of a suture snare captured by a snare enclosure formed by a piercing tip 108 and a curved portion 106 of a passer tube 102, with the snare enclosure in a closed configuration, according to embodiments of the present invention. FIG. 12 illustrates the resilient anchor element 152 released from the snare enclosure with the snare enclosure in an open configuration, according to embodiments of the present invention. FIG. 13 illustrates a snare enclosure retracted back into the closed position after release of the resilient anchor element 152, according to embodiments of the present invention.

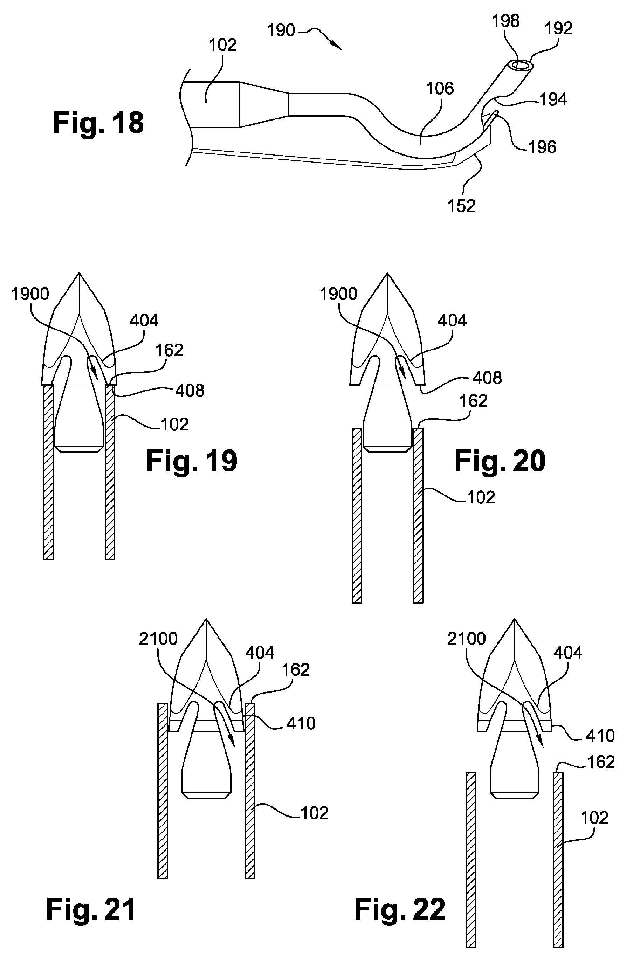

The snare enclosure illustrated in FIGS. 11 through 13 is formed by and/or bounded by the inner portion 412 of hook element 403 and also by the distal end 162 of passer tube 102, according to embodiments of the present invention. In a closed configuration, the bottom end 408 of flange 404 abuts or is in contact with the passer tube 102 as illustrated in FIG. 19 and/or the outer surface 410 of piercing tip 108 abuts or is in contact with the passer tube 102, as illustrated in FIG. 21, according to embodiments of the present invention. As such, the closed configuration captures the suture snare 150, for example the resilient anchor element 152 of suture snare 150, between the hook element 403 and the distal end 162 of passer tube 102, thereby preventing the release or the pulling away of the suture snare 150 from within such a snare enclosure 1900, 2100 when the snare enclosure is in a closed configuration.

In an open configuration of the snare enclosure, the hook elements 403 are separated from distal end 162 by a distance which permits the suture snare 150 and/or resilient anchor element 152 to be passed into and out of the snare enclosure. Such an open configuration is illustrated in FIG. 12, and permits either release of a suture snare 150 that was held within the snare enclosure or capture or hooking of the suture snare 150 with the one or more hook elements 403, according to embodiments of the present invention.

Because the piercing tip 108 is coupled to the actuator rod 202, sliding the actuator rod 202 with respect to the passer tube 102 moves the piercing tip 108 so as to change the snare enclosure between the open and closed configurations, according to embodiments of the present invention. For example, sliding the button 110 of FIG. 3 in a direction opposite arrow 209 slides the piercing tip 108 from the position of FIG. 11 in which the piercing tip 108 abuts the passer tube 102 (e.g. abuts the distal end 162 thereof) to the position of FIG. 12 in which the piercing tip 108 is separated and pushed outwardly from passer tube 102 (e.g. no longer abuts the distal end 162 thereof), according to embodiments of the present invention. As such, such a sliding motion moves the snare enclosure from the closed to the open configuration. Sliding the button 110 of FIG. 3 in the direction of arrow 209 slides the piercing tip 108 in the opposite direction toward the distal end 162, thereby moving the snare enclosure from the open to the closed configuration. According to embodiments of the present invention, the button 110 may simply be released when the snare enclosure is in the open configuration and the spring element 210 will pull the actuator rod 202 and thus the piercing tip 108 back to the position in which the snare enclosure is in the closed configuration. The closed configuration of the snare enclosure, without the snare 150 or resilient anchor member 152 captured thereby, is illustrated in FIG. 13.

FIGS. 14-17 illustrates a suture passer 100 used to pass a suture snare through tissue, according to embodiments of the present invention. FIGS. 14-17 illustrate a procedure in which suture is passed through a labrum tissue 252 which is attached to a glenoid 250 in order to attach it to a capsule tissue 256 element, according to embodiments of the present invention. A cannula 254 may be used to provide a pathway for the suture passer 100 to access the capsule 256, according to embodiments of the present invention. Although a particular procedure is shown and described, one of ordinary skill in the art, based on the disclosure provided herein, will appreciate a number of other similar and/or non-similar procedures in which device 100 may be used.

FIG. 14 illustrates a resilient anchor element 152 of a suture snare being passed through labrum tissue 252 by a suture passer with the resilient anchor element 152 captured by a snare enclosure of the suture passer, according to embodiments of the present invention. At least a portion of the suture snare 150 is captured by the snare enclosure; for example, a portion of the resilient anchor element 152 is captured by the snare enclosure. The tissue may be pierced with piercing tip 108 from a first tissue side 258 to a second tissue side 260 to form a hole, according to embodiments of the present invention. The resilient anchor element 152 may then be passed through the hole from the first side 258 to the second side 260 by passing the snare enclosure through the hole and by deforming the resilient anchor element 152, according to embodiments of the present invention.

Once the resilient anchor element 152 has been passed through the hole from the first side 258 to the second side 260, the resilient anchor element 152 resumes its undeformed or normal shape or state, according to embodiments of the present invention. The snare enclosure may be placed into an open configuration to release the suture snare 150 and/or the resilient anchor element 152, according to embodiments of the present invention. According to embodiments of the present invention, the resilient anchor element 152 is released from the snare enclosure upon opening of the snare enclosure; according to other embodiments of the present invention, the snare enclosure and/or piercing tip 108 is moved away from the resilient anchor element, or turned, in order to cause release of the resilient anchor element 152 from the snare enclosure.

Once the snare enclosure has been moved to the open configuration and the suture snare 150 released, the snare enclosure may be moved back into the closed configuration, and the piercing tip 108 and snare enclosure withdrawn back through the hole in the tissue 252 from the second side 260 to the first side 258, according to embodiments of the present invention. Without the resilient anchor element 152, the piercing tip 108 and/or passer tube 102 might tend to pull the suture snare 150 back through the hole along with the rest of the device 100. However, the resilient anchor element 152 may have at least one dimension in an undeformed state that is larger than a largest dimension of the hole, according to embodiments of the present invention. Thus, the undeformed state of the resilient anchor element 152 holds the resilient anchor element 152 on side 260 as the rest of the piercing tip 108 and passer tube 102 are withdrawn, according to embodiments of the present invention.

As illustrated in FIG. 15, once the resilient anchor element 152 has been passed through tissue 252, and before the suture passer 100 is removed through the cannula 254, the snare enclosure formed by the piercing tip 108 and the passer tube 102 may be used to pick up or capture the resilient anchor element 152 to pull it back up through cannula 254, according to embodiments of the present invention. According to embodiments of the present invention, the hook elements 403 are used to hook the resilient anchor element 152 when the snare enclosure is in the open configuration.

In addition, in a similar manner, multiple passes of the resilient anchor element 152 may be achieved through tissue 252 at different hole locations using the suture passer device 100, from surface 258 to 260 and/or from surface 260 to 258, without withdrawing the device 100 or the piercing tip 108 from within the capsule 256, according to embodiments of the present invention. After the final suture pass, the resilient anchor element 152 may be picked up by the device 100 as illustrated in FIG. 15, and withdrawn through the cannula 254, for example to tie a knot or connect the suture to other tissue, according to embodiments of the present invention.

In addition to one or more passes through the same tissue, the suture passer device 100 may also be used to pass the suture snare 150 one or more passes through a different tissue or a different tissue location. FIG. 16 illustrates an optional passage of the suture passer through capsule tissue 180 and subsequent capture of the resilient anchor element 152 by the snare enclosure of the suture passer 100, according to embodiments of the present invention. The snare enclosure may be placed into the closed configuration, and the piercing tip 108 may be inserted through tissue element 180 from a first side 182 to a second side 184, according to embodiments of the present invention. The snare enclosure may then be placed into the open configuration and used to snag or pick up or hook the resilient anchor element 152, for example the resilient anchor element 152 that has already been passed through labrum tissue 252, as illustrated in FIG. 16, according to embodiments of the present invention.

Once the resilient anchor element 152 has been hooked or snagged or otherwise placed within the snare enclosure, the actuator rod 202 may be moved to place the snare enclosure into the closed position, and the snare enclosure (and resilient anchor member 152) may then be pulled through tissue 180 from second side 184 to first side 182, as illustrated in FIG. 17, according to embodiments of the present invention. The suture 270 may be inserted through the suture capture element 154, either before or after the suture snare 150 is passed through the tissue 252 and/or 182, according to embodiments of the present invention. The suture snare 150 may then be pulled by the snare enclosure of the piercing tip 108 and passer tube 102 out through the cannula 254, thereby causing the suture capture element 154 to pull the suture 270 through the same path, as illustrated in FIG. 17, according to embodiments of the present invention.

Any, or any subset, of these steps may be performed with the same device 100 in the same evolution (for example without withdrawing the device 100 from the gleno-humeral space), according to embodiments of the present invention. The device 100 may permit full operation entirely with a single hand, according to embodiments of the present invention.

FIG. 18 illustrates an alternative suture passer 190 and snare enclosure configuration, according to embodiments of the present invention. Suture passer 190 includes a side opening in a sharpened cannulated tube, in which a sliding gate traps the snare in the side opening, according to embodiments of the present invention. The suture passer 190 includes a passer tube 102 having a curved portion 106; the piercing tip 192 is formed on a distal end 198 of the passer tube 102, according to embodiments of the present invention. The passer tube 102 includes a sidewall opening 194 located on the passer tube 102 proximally of the distal end 198. The sidewall opening 194 may be located on the curved portion 106. The actuator rod comprises a sliding gate 196, and the snare enclosure is formed by the sidewall opening 194 and the sliding gate 196, according to embodiments of the present invention.

FIG. 19 illustrates a piercing tip 108 and a passer tube 102 forming a snare enclosure 1900 in a closed configuration, according to embodiments of the present invention. FIG. 20 illustrates the piercing tip 108 and the passer tube 102 of FIG. 19 forming a snare enclosure 1900 in an open configuration, according to embodiments of the present invention. FIG. 21 illustrates a piercing tip 108 and passer tube 102 forming an alternative snare enclosure 2100 in a closed configuration, according to embodiments of the present invention. FIG. 22 illustrates the piercing tip 108 and passer tube 102 of FIG. 21 forming the alternative snare enclosure 2100 in an open configuration, according to embodiments of the present invention.

According to embodiments of the present invention, the curved portion 106 of the passer tube 102 is curved left, curved right, or extends straight head with respect to the elongated and substantially straight passer tube 102 (for example for use with a hip labrum), according to embodiments of the present invention. According to some other embodiments, a suture snare 150 is not used, and instead the suture 270 tips are configured to attach directly to the piercing tip 108.

Although square, rectangular, and diamond shaped resilient anchor element 152 shapes are described, other shapes may be used; for example, the resilient anchor element 152 may be formed in a different geometric two-dimensional shape, and/or formed into a three-dimensional feature such as, for example, a ferrule, or ball crimped on the end of the suture snare 150, according to embodiments of the present invention.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A system for suture passage through a tissue,
the system comprising:
a suture snare (150) comprising a suture capture element (154); and
a piercing tip (108; 192) configured to form through the tissue a hole connecting a first side and a second side of the tissue,
**characterized in that** the suture snare (150) further comprises a resilient anchor element (152), which has, in an undeformed state of the resilient anchor element, at least one dimension larger than a largest dimension of the hole, and which is configured to deform during insertion of the resilient anchor element through the hole from the first side to the second side of the tissue, then to substantially resume, after passing through the hole, the undeformed state that holds the resilient anchor element (152) on the second side of the tissue.

2. The system of claim 1, **characterized in that** the resilient anchor element (152) is arranged at an end of the suture snare (150), and **in that** the suture capture element (154) is arranged between the two opposed ends of the suture snare (150).

3. The system of any one of the preceding claims, **characterized in that** the resilient anchor element (152) comprises a shape set wire (156) formed into a closed loop in the undeformed state.

4. The system of claim 3, **characterized in that** the closed loop is substantially square-, diamond-, or rectangle- shaped in the undeformed state.

5. The system of any one of the preceding claims, **characterized in that** the resilient anchor element (152) is formed of a shape memory material, especially formed of nitinol wire.

6. The system of any one of the preceding claims, **characterized in that** the system further comprises:
a passer tube (102) which is substantially rigid;
an actuator rod (202); and
a snare enclosure (1900; 2100) formed by the passer tube and the actuator rod, and **in that** the actuator rod is movable to move the snare enclosure between a closed configuration in which the snare enclosure completely captures a portion of the suture snare (150) and an open configuration in which the snare enclosure does not completely capture the portion of the suture snare and permits release of the suture snare from the snare enclosure.

7. The system of claim 6, **characterized in that** the piercing tip (192) is formed on a distal end (198) of the passer tube (102), **in that** the passer tube (102) comprises a sidewall opening (194) located on the passer tube proximally of the distal end, **in that** the actuator rod (102) comprises a sliding gate (196), and **in that** the snare enclosure is formed by the sidewall opening and the sliding gate.

8. The system of claim 7, **characterized in that** the passer tube (102) comprises a curved portion (106) adjacent to the distal end (198).

9. The system of claim 8, **characterized in that** the sidewall opening (194) is located on the curved portion (106).

10. The system of claim 6, **characterized in that** the actuator rod (202) comprises the piercing tip (108).

11. The system of claim 10, **characterized in that** the passer tube (102) comprises a distal end (162), **in that** the piercing tip (108) extends from the distal end (162), and **in that** the piercing tip comprises one or more hook elements (403).

12. The system of claim 11, **characterized in that** the snare enclosure (1900; 2100) is formed by the passer tube (102) and the one or more hook elements (403), **in that** the one or more hook elements abut the passer tube when the snare enclosure is in the closed configuration, and **in that** the one or more hook elements do not abut the passer tube when the snare enclosure is in the open configuration.

13. The system of claim 12, **characterized in that** the passer tube (102) further comprises a proximal end, and **in that** the actuator rod (202) is slidable within the passer tube to move the snare enclosure (1900; 2100) between the open and closed configurations, such that sliding the actuator rod in a direction from the proximal end toward the distal end (162) of the passer tube moves the one or more hook elements (403) away from the distal end, and sliding the actuator rod in a direction from the distal end toward the proximal end moves the one or more hook elements into engagement with the passer tube.

14. The system of claim 13, **characterized in that** the system further comprises a handle portion (104) rigidly coupled with the passer tube (102), the handle portion comprising an actuation mechanism (206, 208, 210, 212, 214) coupled with the actuator rod (202), such that movement of the actuation mechanism slides the actuator rod with respect to the passer tube.

15. The system of any one of claims 11 to 14, **characterized in that** the passer tube (102) comprises a curved portion (106) adjacent to the distal end (162).
